# EUROPEAN PATENT APPLICATION

(11) **EP 0 788 791 A1**
(43) Date of publication of application: **13.08.1997**
(21) Application number: 96927897.7
(22) Date of filing: 23.08.1996
(51) Int. Cl.: A61K 9/46, A61K 47/10

(54) **LOW-PRESSURE TABLETED EFFERVESCENT PREPARATION**

(30) Priority: 07.09.1995 JP 229802/95; 17.05.1996 JP 123154/96
(71) Applicant: OTSUKA PHARMACEUTICAL CO., LTD., Chiyoda-ku Tokyo 101 (JP)
(72) Inventor: TAKAICHI, Akihisa 172-3, Aza Nakajima Takashima, Tokushima 772 (JP); OKAMOTO, Toshihiko, Tokushima 772 (JP); MATSUMOTO, Toshiaki, Tokushima 772 (JP)
(74) Representative: Hansen, Bernd, Dr. Dipl.-Chem.
(86) International application number: JP9602372
(87) International publication number: WO9709037

(57) **Abstract**

The present invention provides a low-pressure-tableted effervescent preparation which is satisfactory in tablet hardness and dissolution properties, the preparation comprising 10 to 35% by weight of sodium hydrogencarbonate and/or sodium carbonate, 10 to 70% by weight of a neutralizing agent and 0.02 to 40% by weight of at least one member selected from the group consisting of maltitol, sorbitol, maltose, trehalose, mannitol, lactitol and xylitol.

## Description

### Technical Field

The present invention relates to a low-pressure-tableted effervescent preparation.

### Prior Art

Effervescent tablets are produced using a tableting machine. When specific kinds or various kinds of components are used, it is sometimes necessary to carry out the tableting under high pressure in order to obtain tablets of sufficient hardness for distribution. However, when high pressure is employed for tableting, problems with the durability of the machine occur due to the extra load. In addition, the high pressure tableting induces problems such as binding, capping, sticking of the tablets, increasing the likelihood of trouble during production.

Therefore, effervescent tablets are preferably produced under low pressure. However, at present, no technique has been developed for producing desired effervescent tablets under lower pressure.

### Disclosure of the Invention

An object of the present invention is to provide an effervescent preparation produced under low pressure.

The present inventors conducted extensive research and found that the foregoing object can be achieved when a specific amount of at least one member selected from the group consisting of maltitol, sorbitol, maltose, trehalose, mannitol, lactitol and xylitol is used for the production of an effervescent preparation. The present invention has been accomplished based on this finding.

The present invention provides a low-pressure-tableted effervescent preparation comprising 10 to 35% (by weight, the same applies hereinafter) of sodium hydrogencarbonate and/or sodium carbonate, 10 to 70% of a neutralizing agent and 0.02 to 40% of at least one member selected from the group consisting of maltitol, sorbitol, maltose, trehalose, mannitol, lactitol and xylitol. In particular, the present invention provides said low-pressure-tableted effervescent preparation comprising maltitol.

The present invention also provides said low-pressure-tableted effervescent preparation further comprising the following vitamins in the following proportions, i.e., an effervescent multivitamin preparation.

| Component | Proportion (% by weight/preparation) |
|---|---|
| Vitamin A | 0.0012 - 0.06 |
| Vitamin B₁ | 0.002 - 0.4 |
| Vitamin B₂ | 0.003 - 0.3 |
| Vitamin B₆ | 0.004 - 0.4 |
| Vitamin B₁₂ | 0.000012 - 0.0012 |
| Pantothenic acid | 0.02 - 1.0 |
| Niacin | 0.034 - 1.7 |
| Vitamin C | 0.1 - 20 |
| Vitamin D | 0.000005 - 0.0025 |
| Vitamin E | 0.016 - 1.6 |
| Folic acid | 0.0008 - 0.08 |
| Biotin | 0.0006 - 0.12 |
| β-Carotin | 0.002 - 0.6 |

According to the present invention, the use of said specific components enables the production of the desired effervescent tablets under lower pressure, the tablets having higher hardness, shorter dissolution time and excellent tablet properties. Further, the proportions of the above specific components can be suitably varied within the specified range to provide the optimum tableting conditions for the tableting machine to be used, and to obtain tablets with desired properties. Moreover, low-pressure tableting reduces the load on the machine and enables smoother production with reduced likelihood of troubles in tableting (binding, capping and sticking).

The preparation of the present invention will be described below in more detail. An essential component of said preparation is selected from maltitol, sorbitol, maltose, trehalose, mannitol, lactitol and xylitol. These materials have properties typically required of excipients of effervescent tablets, i.e., (1) high solubility in water, (2) good taste, (3) ease of handling (high flowability and low moisture absorption), etc. In addition, they can impart excellent properties to the tablets, as described above.

Said specific components can be used in place of a part or the whole of the purified sucrose conventionally used as an excipient. Thus, the amount of said component can be suitably selected from the range in which conventional excipients are used. In particular, for the contemplated low-pressure tableting according to the present invention, it is necessary to use said component in a proportion of at least 0.02%, preferably about 0.5 to about 20%.

The use of said specific component (hereinafter sometimes referred to as "excipient") is essential to the present invention, but does not inhibit the use of other excipients. Optional excipients can be used which include excipients conventionally used, such as purified sucrose, starch, lactose, sucrose, glucose, calcium phosphate, calcium sulfate, and the like.

The essential and optional excipients for use in the present invention may be in the form of a conventionally available crystalline powder or granule. The particle size of the powder or granule is not limited specifically, but generally an average particular size of 100 to 250 µm is preferred.

The other essential components of the effervescent preparation of the invention are a specific amount of sodium hydrogencarbonate and/or sodium carbonate as the effervescent component and a neutralizing agent. The term "neutralizing agent" used herein refers to an acidic compound capable of producing carbon dioxide gas by neutralization of sodium hydrogencarbonate and/or sodium carbonate. Typical examples of such acidic compounds include L-tartaric acid, citric acid, lactic acid, dl-malic acid, fumaric acid, L-ascorbic acid and the like.

The proportion of the effervescent component in the preparation of the invention is 10 to 35%, preferably 20 to 30% of sodium hydrogencarbonate and/or sodium carbonate, and the proportion of the neutralizing agent is 10 to 70%, preferably 10 to 40%. In particular, a suitable proportion of sodium hydrogencarbonate is 10 to 35%, preferably 20 to 30%, and a suitable proportion of sodium carbonate is 11 to 31%, preferably 20 to 26%. It is the most desirable to use sodium hydrogencarbonate alone in a proportion selected from the range of 20 to 25%. The neutralizing agent is used preferably in at least equivalent amount relative to sodium hydrogencarbonate.

The presence of the effervescent component and neutralizing agent in specific amounts makes the preparation of the invention highly soluble in water. A further advantage is that the solution of the preparation in water shows good taste. However, if the effervescent component and neutralizing agent are used in proportions outside said specific ranges, it would disadvantageously become difficult to produce a product satisfactory in effervescent property and taste.

Said effervescent component in a conventionally available powder form can be used in the present invention. The particle size (particle diameter, dimension, etc.) is not specifically limited, but preferably 250 to 350 µm on average. Generally, sodium hydrogencarbonate and sodium carbonate having a particle diameter of 100 to 150 µm are preferred.

The effervescent preparation which is capable of achieving the contemplated results of the invention can be obtained by using the effervescent component and excipients in the proportions within the specified ranges.

The effervescent preparation of the invention, which essentially contains the predetermined amounts of the specific components, may further contain additives conventionally used. Useful additives include, for example, thickeners, surfactants (emulsifiers), osmotic pressure regulators, electrolytes, sweeteners, flavors, pigments, pH adjusters, etc. The proportions of these additives can be suitably selected, usually from the range which can produce the inherent effects of respective additives. Within said range, the additives do not adversely affect the contemplated results of the invention.

The effervescent preparation of the invention may also contain powders containing antioxidants such as carotin, vitamin E, catechin and the like. In particular, remarkably good results can be achieved when powders containing carotin, vitamin E and the like are employed, since such powders have effects of adjusting markedly deteriorated tableting properties and preventing the matrix of the fat-soluble antioxidant from being destroyed by high pressure tableting.

The carotin is a precursor of vitamin A and has a provitamin A activity. Useful carotins include α-, β-and γ-carotins. The vitamin E can be any of α-, β-, γ-and δ-tocopherols. Said carotin and vitamin E may be either natural or synthetic for use in the invention. Typical examples of catechins are epigallocatechin gallates (EGCg), epicatechin gallates (ECg), epigallocatechins (EGC) and epicatechins (EC), such as tea polyphenol, apple polyphenol and the like.

Among the antioxidant-containing powders, powders containing carotin, vitamin E or like fat-soluble substances include, for example, those having a matrix formed from said antioxidant dissolved in a suitable fat and oil and a suitable base material (excipient) for forming particles, or an emulsifier or the like for solubilization. Examples of useful fats and oils include conventional fats and oils such as corn oil, peanut oil, essential oil and like vegetable fats and oils. Examples of base materials (excipients) for forming particles include conventional materials including saccharides such as lactose, dextrin, etc.; polysaccharides capable of functioning also as an emulsifier, such as gum arabic, casein, etc.; and proteins. Examples of useful emulsifiers are lecithin, glycerin fatty acid ester, sucrose fatty acid ester, calcium stearoyl lactate, sorbitan fatty acid ester, propyleneglycol fatty acid ester, etc. The antioxidant-containing powder may contain an oxidation inhibitor for inhibiting the oxidation of carotin or like antioxidants, such as L-ascorbic acid palmitic acid ester, vitamin C, enzymatically modified rutin, etc. The incorporation of such oxidation inhibitor in the powder is desirable.

The matrix of the antioxidant-containing powder can be formed by conventional methods such as spray drying to give the desired powder having a suitable particle size.

Among the antioxidant-containing powders, powders containing catechin or like water-soluble antioxidant can be easily prepared merely, for example, by extracting the antioxidant with hot water or alcohol, concentrating the extract, and making the concentrate into particles by conventional methods such as spray drying.

The proportion of the antioxidant-containing powder in the preparation of the invention is not greater than 15%, preferably 0.1 to 5.0%. The proportion of the antioxidant (fat-soluble substance) in the powder is 0.2 to 20%, preferably 0.5 to 10%. This means that the balance 99.8 to 80% consists of the fat and oil, the base material for forming particles or emulsifier, and the oxidation inhibitor optionally added with these components. The oxidation inhibitor, when incorporated in the powder, is preferably used in a proportion of about 0.5 to about 20% of the antioxidant-containing powder. The proportion of catechin or like water-soluble antioxidant in the powder is at least 10%.

The effervescent preparation of the present invention may further comprise, in addition to the above essential components and optional additives, predetermined amounts of various vitamins to obtain an effervescent multivitamin preparation. The species and proportions of the vitamins to be used are as described above. Among these vitamins, vitamin A, vitamin D, vitamin E and β-carotin are incorporated into the preparation of the invention preferably in the form of a vitamin-containing powder, like the antioxidant-containing powder mentioned above. The reason is that such powder is uniformly dispersed in cold water (without floatation or precipitation) and less susceptible to deterioration caused by oxidization, since said powder has relatively good barrier property against oxygen.

Various kinds of said vitamin-containing powders are commercially available, and any of them can be employed in the present invention.

Thus, the effervescent multivitamin preparation of the present invention contains vitamins in suitable amounts according to the standard for daily quota of vitamins (RDI), within the range which can provide a commercially valuable product and maintain the characteristics (tablet properties, taste, etc.) of the product. Said multivitamin preparation of the invention can effectively supply vitamins.

The effervescent multivitamin preparation of the invention is advantageous in that it can supply vitamins with better taste than vitamin supplements to be taken with water, and can keep the vitamin components stable for a longer period than vitamin solutions, since the preparation of the invention is in a dry form. The incorporation of a plurality of vitamin components usually results in deterioration of the tableting properties (increase in pressure required for tableting, lower tablet hardness, longer dissolution time, etc.). The preparation of the present invention is advantageous also in that the amount of the specific components (such as maltitol) can be suitably selected to provide the optimum production conditions (such as suitability for the tableting machine) and the optimum tablet properties.

The effervescent preparation of the invention can be prepared by conventional methods using said essential components and optional additives. The conventional methods include a dry (direct) tableting process which comprises simply mixing the starting materials and tableting the mixture, and a wet tableting process which comprises wetting a part of the starting materials, kneading all of the starting materials, drying and sieving the kneaded mixture to form granules, and tableting the granules. The dry tableting process can be carried out easily, but has a drawback that when components are used which are likely to be modified by the reaction with each other, the resulting tablets have a problem with long-term storage stability. On the other hand, the wet tableting process, which is free from the above problem, can be carried out under lower pressure than the dry tableting process to produce tablets of the equivalent hardness. However, the wet tableting process disadvantageously involves troublesome procedures. Therefore, the effervescent tablets of the present invention can be prepared by a method suitably selected from the dry tableting process and wet tableting process depending on the kinds and proportions of the components. The wet tableting process is advantageous in that the tableting can be carried out under satisfactorily low pressure using the excipients, i.e., maltitol, sorbitol, maltose, trehalose, mannitol, lactitol and xylitol in a small amount within the above-specified range. The pressure for tableting is usually selected from the range of about 0.2 to about 0.35 ton/cm², preferably about 0.24 to about 0.31 ton/cm². It is recommendable that the pressure in said range result in tablets having hardness of about 4.0 to about 6.6 kp, preferably about 4.5 to about 5.9 kp. The hardness of the tablets is expressed in terms of a pressure under which the tablet was broken by compression of two sides at a specific loading speed (1.0 mm/sec) using a Schleuniger tablet hardness meter, Model 4M (Schleuniger-4M).

### Best Mode for Carrying Out the Invention

Given below are Test Examples and Examples to clarify the present invention in more detail. The parts in the Examples are all by weight.

### Test Example 1

The excipient used was 1900 mg of purified sucrose or 1900 mg of a mixture of purified sucrose and maltitol wherein the proportions were varied as shown in Table 1. To a mixture of said excipient, 500 mg of L-ascorbic acid and 1200 mg of L-tartaric acid were added 1200 mg of sodium hydrogencarbonate, a suitable amount of sweetener and multivitamin and a trace of flavor and pigment. The mixture was tableted to obtain effervescent tablet samples having the same hardness. Using the samples, the effects of maltitol on the tableting properties of effervescent tablets were tested.

The pressure for tableting was measured with KIKUSUI PDP-1, and the dissolution time was determined by dissolving the samples in 140 cc of cold water (8°C).

The results are shown in Table 1.

**Table 1**

| Sample No. | Purified sucrose | Maltitol | Pressure for tableting | Dissolution time |
|---|---|---|---|---|
| 1 | 1900 mg | 0 mg | 3.9 t | 2 min. 10 sec. |
| 2 | 1600 mg | 300 mg | 3.0 t | 2 min. 00 sec. |
| 3 | 1300 mg | 600 mg | 2.0 t | 1 min. 44 sec. |
| 4 | 1000 mg | 900 mg | 1.6 t | 1 min. 27 sec. |

Table 1 shows that the larger the amount of maltitol is, the lower the pressure for making tablets of the same hardness becomes (the more successfully the low-pressure tableting can be achieved) and the shorter the dissolution time becomes.

### Test Example 2

Effervescent tablet samples having the same hardness were prepared from the same components as in Test Example 1 with the exception of varying the proportions of purified sucrose and maltitol as shown in Table 2 and employing a wet tableting process. Using the samples, the effects of maltitol on the tableting properties of effervescent tablets were tested.

The pressures for tableting and dissolution time were measured in the same manner as in Test Example 1.

Table 2 shows the results.

**Table 2**

| Sample No. | Purified sucrose | Maltitol | Pressure for tableting | Dissolution time |
|---|---|---|---|---|
| 1 | 1900 mg | 0 mg | 3.6 t | 1 min. 53 sec. |
| 2 | 1895 mg | 5 mg | 2.9 t | 1 min. 48 sec. |
| 3 | 1890 mg | 10 mg | 2.6 t | 1 min. 46 sec. |
| 4 | 1870 mg | 30 mg | 2.3 t | 1 min. 43 sec. |
| 5 | 1850 mg | 50 mg | 1.9 t | 1 min. 42 sec. |
| 6 | 1800 mg | 100 mg | 1.8 t | 1 min. 42 sec. |
| 7 | 1600 mg | 300 mg | 1.6 t | 1 min. 36 sec. |

As is apparent from the above, if tablets are made by a wet tableting process which comprises mixing the maltitol powder with the other components and granulating the mixture by addition of a binding solution or by a wet tableting process using maltitol (powder or syrup) as dissolved in the binding solution, the desired low-pressure tableting and short dissolution time can be achieved using maltitol in a smaller amount than in the dry tableting process.

### Example 1

### Wet tableting process

| | |
|---|---|
| Granulated sugar | 1900 mg |
| Maltitol | 3 mg |
| L-Ascorbic acid | 500 mg |
| L-Tartaric acid | 1200 mg |
| Sodium hydrogencarbonate | 1200 mg |
| Sweetener | q.s. |
| Vitamins | Predetermined amount |
| Flavor and pigment | Trace |
| Total | 5100 mg |

As the vitamins, the multivitamins A to E of the following compositions were used.

**Table 3**

| Component | A | B | C | D | E |
|---|---|---|---|---|---|
| Vitamin A powder | 0.03 | 0.06 | 0.45 | 0.2 | 0.2 |
| Vitamin B₁ | 0.01 | 0.8 | 0.05 | 0.5 | 0.02 |
| Vitamin B₂ | 0.03 | 0.3 | 0.2 | 0.1 | 0.03 |
| Vitamin B₆ | 0.04 | 0.2 | 0.1 | 0.08 | 0.04 |
| Vitamin B₁₂ | 0.00003 | 0.0012 | 0.0001 | 0.0003 | 0.0001 |
| Pantothenic acid | 0.05 | 0.8 | 0.5 | 0.4 | 0.2 |
| Niacin | 0.1 | 0.8 | 0.5 | 0.3 | 0.4 |
| Vitamin C | 0.1 | 0.8 | 0.5 | 0.3 | 10 |
| Vitamin D powder | 0.01 | 0.18 | 0.1 | 0.05 | 0.02 |
| Vitamin E powder | 0.3 | 0.8 | 1.0 | 0.5 | 0.4 |
| Folic acid | 0.001 | 0.08 | 0.88 | 0.01 | 0.008 |
| Biotin | 0.001 | 0.21 | 0.01 | 0.1 | 0.006 |
| β-carotin powder | 0.3 | 1.0 | 1.0 | 0.6 | 1.0 |

The β-carotin powder in the multivitamins was prepared by forming a matrix from 4 parts of β-carotin dissolved in 32 parts of vegetable fat and oil, 2 parts of L-ascorbic acid, 2 parts of tocopherol extract, 0.9 part of tea extract (containing tea polyphenol), 0.1 part of L-ascorbic acid palmitic acid ester and, as the base materials, 58 parts in total of gum arabic and saccharides.

A granulating liquid consisting of maltitol syrup, water and ethyl alcohol was added to a mixture of the above specified amounts of ascorbic acid, tartaric acid and granulated sugar to prepare granules, and the other components were admixed with the granules. The mixture was formed into effervescent tablets of the invention under a pressure of 0.27 ton/cm².

The tablets thus obtained (containing multivitamin E shown in Table 3) had hardness of 4.5 kp and dissolution time of 1 minute and 40 seconds.

The hardness and the dissolution time of the tablets were measured by the above methods.

As other stability test items, the swelling of the packaging material (by visual inspection), discoloration of tablets (using a color difference meter, product of Tokyo Denshoku Co., Ltd., Color Ace Model TC-1), and the variation of taste (by a functional test) were tested and evaluated.

The packaging material showed no swelling after 2-week storage at 50°C. The tablets exhibited a discoloration of ΔE value <3, and the variation of taste was within the allowable range.

### Examples 2 to 7

The effervescent tablets of the present invention were prepared from the components shown in Table 4 below (the numerical values in Table 4 are by % by weight). Examples 2 to 4 were carried out by the dry (direct) tableting process, and Examples 5 to 7 by the wet tableting process.

**Table 4**

| Example No. | 2 | 3 | 4 | 5 | 6 | 7 |
|---|---|---|---|---|---|---|
| Granulated sugar | - | 20 | 26 | 35 | 37 | 35 |
| Maltitol | 38 | 18 | 13 | 6 | 2 | 9 |
| L-Ascorbic acid | 10 | 10 | 11 | - | - | - |
| L-Tartaric acid | 24 | - | - | 33 | - | - |
| Citric acid | - | 24 | - | - | 32 | - |
| Malic acid | - | - | 25 | - | - | 30 |
| Sodium hydrogencarbonate | 24 | 20 | 18 | 16 | 23 | 21 |
| Sodium carbonate | - | 4 | - | 2 | - | 2 |
| Calcium carbonate | - | - | 3 | 3 | - | - |
| Potassium carbonate | - | - | - | - | 0.6 | 0.4 |
| Sweetener | q.s. | q.s. | q.s. | q.s. | q.s. | q.s. |
| Multivitamin | A-E | A-E | A-E | A-E | A-E | A-E |
| Flavor/Pigment | Trace | Trace | Trace | Trace | Trace | Trace |
| Total (g/tablet) | 5.0 | 5.1 | 5.2 | 4.9 | 4.8 | 5.1 |

The multivitamins A to E shown in Table 3 were used in each of the above Examples. Thus, in each Example, five kinds of tablets were prepared from the multivitamins A to E, respectively.

### Examples 8 to 13

The effervescent tablets of the present invention were prepared from the components shown in Table 5 below (numerical values in Table 5 are by % by weight), by the wet tableting process using maltitol as dissolved in the binding solution.

**Table 5**

| Example No. | 8 | 9 | 10 | 11 | 12 | 13 |
|---|---|---|---|---|---|---|
| Granulated sugar | 37 | 40 | 34 | 37 | 37 | 38 |
| Maltitol | 0.6 | 0.2 | 0.06 | 0.6 | 0.2 | 0.06 |
| L-Ascorbic acid | 10 | 10 | 10 | 10 | - | 10 |
| L-Tartaric acid | 3 | 13 | 13 | - | 5 | 24 |
| Citric acid | 14 | 12 | - | 12 | 16 | - |
| Malic acid | 11 | - | 13 | 12 | 13 | - |
| Sodium hydrogencarbonate | 18 | 16 | 25 | 23 | 24 | 24 |
| Sodium carbonate | - | 2 | - | - | - | - |
| Calcium carbonate | 3 | 2 | - | - | - | - |
| Potassium carbonate | 0.4 | 0.4 | - | - | - | - |
| Sweetener | q.s. | q.s. | q.s. | q.s. | q.s. | q.s. |
| Multivitamin | A-E | A-E | A-E | A-E | A-E | A-E |
| Flavor/Pigment | Trace | Trace | Trace | Trace | Trace | Trace |
| Total (g/tablet) | 5.0 | 4.9 | 4.8 | 5.2 | 5.1 | 5.0 |

The multivitamins A to E shown in Table 3 were used in each of the above Examples. Thus, in each Example, five kinds of tablets were prepared from the multivitamins A to E, respectively.

### Possibility of Industrial Application of the Invention

According to the present invention, an epoch-making effervescent preparation is provided by low pressure tableting, the preparation having satisfactory tablet hardness and shorter dissolution time. The preparation of the invention is useful in the food field and the pharmaceutical field.

## Claims

1. A low-pressure-tableted effervescent preparation comprising 10 to 35% by weight of sodium hydrogencarbonate and/or sodium carbonate, 10 to 70% by weight of a neutralizing agent and 0.02 to 40% by weight of at least one member selected from the group consisting of maltitol, sorbitol, maltose, trehalose, mannitol, lactitol and xylitol.

2. The low-pressure-tableted effervescent preparation according to claim 1 which comprises maltitol.

3. The low-pressure-tableted effervescent preparation according to claim 1 or 2 which further comprises the following vitamins in the following amounts.
| Component | Proportion (% by weight/preparation) |
|---|---|
| Vitamin A | 0.0012 - 0.06 |
| Vitamin B₁ | 0.002 - 0.4 |
| Vitamin B₂ | 0.003 - 0.3 |
| Vitamin B₆ | 0.004 - 0.4 |
| Vitamin B₁₂ | 0.000012 - 0.0012 |
| Pantothenic acid | 0.02 - 1.0 |
| Niacin | 0.034 - 1.7 |
| Vitamin C | 0.1 - 20 |
| Vitamin D | 0.000005 - 0.0025 |
| Vitamin E | 0.016 - 1.6 |
| Folic acid | 0.0008 - 0.08 |
| Biotin | 0.0006 - 0.12 |
| β-Carotin | 0.002 - 0.6 |
